# EUROPÄISCHE PATENTSCHRIFT

(11) **EP 2 793 737 B1**
(45) Veröffentlichungstag und Bekanntmachung des Hinweises auf die Patenterteilung: **16.09.2020**
(21) Anmeldenummer: 12813357.6
(22) Anmeldetag: 21.12.2012
(51) Int. Cl.: A61C 13/34

(54) **VERFAHREN ZUM HERSTELLEN UND TRAGPLATTE ZUR AUFNAHME EINES ZAHNTECHNISCHEN MODELLS**
METHOD FOR MANUFACTURING A DENTAL WORKING MODEL AND SUPPORTING FRAME FOR A DENTAL WORKING MODEL
PROCÉDÉ DE FABRICATION D'UN MODÈLE DE TRAVAIL DENTAIRE ET
BASE POUR MODÈLE DE TRAVAIL DENTAIRE

(30) Priorität: 23.12.2011 DE 102011057029
(43) Veröffentlichungstag der Anmeldung: 29.10.2014
(73) Patentinhaber: DENTSPLY SIRONA Inc., York, PA 17401-2991 (US)
(72) Erfinder: GEBHARDT, Andreas, 63505 Langenselbold (DE); HAIZMANN, Martin, 63695 Glauburg (DE); HÖRHOLD, Heiner, 63654 Büdingen (DE); HOCK, Elmar, 63776 Mömbris (DE); VÖLKL, Lothar, 63773 Goldbach (DE); FECHER, Stefan, 63867 Johannesberg (DE)
(74) Vertreter: Stoffregen, Hans-Herbert
(86) Internationale Anmeldenummer: PCT/EP2012/076598
(87) Internationale Veröffentlichungsnummer: WO 2013/092980

(56) Entgegenhaltungen:
- EP-A1- 0 200 193
- WO-A1-2011/103879
- DE-U1-202004 001 768
- FR-A1- 2 715 826
- US-A1- 2010 152 873

## Beschreibung

Die Erfindung bezieht sich auf ein Verfahren zum Herstellen eines in eine eine Referenzierung aufweisende Ausnehmung einer Halterung lösbar einzusetzenden zahntechnischen Modells, wobei die Ausnehmung eine Referenzierung aufweist und das Modell zumindest ein in die Ausnehmung einzusetzendes Zahnteil mit einer Basis aufweist, und wobei unter Verwendung von digitalisierten Daten eines Kiefers oder eines Abschnitts von diesem das zumindest eine Zahnteil hergestellt wird, indem zuvor die digitalisierten Daten in einem Rechner abgespeichert und unter Verwendung dieser einer Bearbeitungsmaschine zur Herstellung von zumindest dem einen Zahnteil zugeführt werden.

Auch nimmt die Erfindung Bezug auf eine Tragplatte zur Aufnahme von einem aus mehreren Segmenten bestehenden Gebissmodell, wobei jedes Segment eine Basis mit einem Zahnteil oder mehreren Zahnteilen aufweist, und wobei die Tragplatte eine Ober- und Unterseite und eine einem Bogen wie Gebissbogen folgende Ausnehmung bzw. Aussparung mit Seitenflächen aufweist, die bzw. von denen zumindest eine der Seitenflächen eine mechanische Referenzierung aufweisen bzw. aufweist, die eine erste Führung für die Basis bildet, wobei die Basis und das Zahnteil als Einheit aus demselben Material hergestellt sind..

Gegenstand der Erfindung ist auch eine zahntechnische Einrichtung umfassend ein zahntechnisches Modell bestehend aus zumindest zwei Segmenten sowie eine das zahntechnische Modell lösbar in einer Ausnehmung bzw. Aussparung aufnehmende
Tragplatte, wobei jedes Segment zumindest ein von einer Basis ausgehendes Zahnteil, vorzugsweise zumindest zwei Zahnteile, aufweist, und das Zahnteil und die Basis als Einheit aus demselben Material hergestellt sind.

Um einen Zahnersatz herzustellen, wird nach einer weit verbreiteten Technik zunächst von einem Gebiss bzw. einem Teil eines solchen nach zuvor erfolgter zahnärztlicher Präparation ein Abdruck hergestellt, der die negative Situation im Mund eines Patienten wiedergibt. Von dem so gebildeten Negativmodell wird ein Positivmodell hergestellt, das den Bereich des Kiefers umfasst, der mit einem Zahnersatz zu versehen ist. Bei ein oder mehreren Zahnstümpfen wird auch die Kieferumgebung mit abgeformt.

Um auf der Basis eines entsprechenden Meistermodells einen Zahnersatz herzustellen, kann eine ausschließlich manuelle Fertigung oder eine solche erfolgen, in dem der in Frage kommende Bereich gescannt und die so erhaltenen digitalisierten Daten zur Herstellung von Zahnersatz im CAD/CAM-Verfahren benutzt werden. Dabei ist es erforderlich, dass entweder zuvor das als Duplikat zu bezeichnende Modell in Duplikatabschnitte zertrennt wird, die sodann einzeln gescannt werden. Um die Duplikatabschnitte einander zuordnen zu können, ist es des Weiteren erforderlich, dass eine Korrelation zum gesamten Duplikat erfolgt. Somit müssen sowohl das Duplikat an sich als auch die Duplikatabschnitte gescannt werden (EP-B-0 913 130).

Um diese Nachteile zu vermeiden, ist es nach der EP-B-1 691 712 bekannt, ohne Trennen des Duplikats dieses zu scannen, wobei die einzelnen Scanns auf Grund einer an dem Duplikat vorhandenen Referenzierung einander zugeordnet werden.

Der WO-A-2010/099959 ist ein zahntechnisches Modell zu entnehmen, das aus einem Basisteil mit Ausnehmungen und aus in diese einzusetzenden Zahnteilen besteht. Um zu vermeiden, dass Zahnteile fehlerhaft in das Basisteil eingesetzt werden, weisen die Zahnteile Orientierungsfortsätze auf.

Um ein Meistermodell herzustellen, wird nach der WO-A-98/10709 eine Trägerplatte mit Ausnehmungen benutzt, die von Seitenflächen mit einer Strukturierung begrenzt ist. Im Bodenbereich der Tragplatte sind Bohrungen vorhanden, in die Stifte von Segmenten des Modells einsetzbar sind, um eine ordnungsgemäße Positionierung der Segmente sicherzustellen.

Eine entsprechende Technik ist auch der WO-A-01/06945 zu entnehmen. Dieser ist eine Tragplatte mit mittels Rapid-Prototyping hergestellten Zahnkränzen zu entnehmen. Zum Fixieren der Zahnkränze weist die Tragplatte eine von Führungsrillen begrenzte durchgehende Aussparung auf, um durch die Aussparung hindurch Sockelmaterial zum Fixieren der Zahnkranzunterseite durchgießen zu können.

Der WO-A-2007/117239 ist ein Verfahren zum automatischen Herstellen einer Form für einen Zahnersatz zu entnehmen. Hierzu wird intraoral der in Frage kommende Kieferbereich gescannt. Dabei wird eine Zahnbasis gebildet, die eine Öffnung zur Aufnahme eines vorgefertigten Abutments aufweist.

In der WO-A-2011/103879 ist ein computerimplementiertes Verfahren zur Erzeugung eines virtuellen Modells eines Satzes von Zähnen beschrieben, um auf deren Basis ein physisches Modell herzustellen.

Eine Tragplatte für ein Modell ist der US-A-5,506,095 zu entnehmen. Die Tragplatte weist eine einem Kieferbogen folgende Aussparung mit Innenseiten auf, die als Referenzierung dienen, um ein Wiedereinsetzen einer in der Aussparung ausgehärteten Form zu ermöglichen. Der Boden weist einen Schlitz auf, in den ein insbesondere aus Kunststoff bestehendes Element einsetzbar ist, um eine Zwischenlage zu dem Modell zu bilden und ein leichtes Einsetzen und Entfernen aus der Tragplatte zu ermöglichen.

Gegenstand der DE-B-10 2004 054 876 ist eine Vermessungseinrichtung zur 3D-Vermessung von Zahnmodellen. Hierzu wird ein Zahnmodell in verschiedenen Rastpositionen aufgenommen, die bekannt sind. Hierdurch wird ein Matchen der einzelnen Aufnahmen erleichtert.

Eine Form zur Herstellung eines Zahnmodells ist aus der DE-C-197 22 989 bekannt. Diese umfasst eine Modellplatte sowie einen einen Zahnabdruck tragenden Abdrucklöffel. Der Hohlraum zwischen Abdruckform und Modellplatte wird mit einem Abformmaterial ausgefüllt. Von diesem gehen Abschnitte von Stiften aus, die in entsprechende Öffnungen der Modellplatte einsetzbar sind.

In der EP-A-1 520 551 wird ein Arbeitstisch für Zahntechniker und Zahnärzte beschrieben, um eine Implantat-Bohrschablone zu erstellen.

Ein Verfahren zur Erzeugung eines Computermodells eines Gebisses auf der Basis digitaler Informationen ist Gegenstand der US-A-2008/0085489.

Eine Führungsschablone zur Herstellung von Zahnersatz ist der WO-A-2008/117323 zu entnehmen.

Die WO-A-2002/39056 bezieht sich auf eine Oberflächenerfassungs- und -erzeugungseinrichtung.

Um einen Zahnersatz in CAD/CAM Technologie herzustellen, sieht die DE-A-10 2005 009 549 vor, dass ein in einem herkömmlichen Verfahren hergestelltes zahntechnisches Modell in einer Grundplatte mittels z. B. Gips oder Klebstoff fixiert wird. Um sodann aus dem Modell geschnittene Segmente zu scannen und die Scandaten zu matchen, weist die Grundplatte eine durch eine Wellengeometrie gebildete Referenzierung auf.

Dem Stand der Technik ist auch ein Verfahren zu entnehmen, bei dem intraoral ein Kieferbereich gescannt und auf der Basis dieser Daten ein zahntechnisches Modell hergestellt wird, wobei Segmente dieses über Stifte in einer Lochrasterplatte fixiert werden. Um ein Vertauschen der Segmente auszuschließen, wird der Lochrasterplatte, auf der die Segmente anzuordnen sind, ein Aufbauplan beigefügt. Dies führt zu einer aufwendigen Handhabbarkeit und stellt letztendlich ein Fehlbelegen der Lochrasterplatte nicht sicher.

Der US-A-2010/0152873 ist ein Verfahren zum Herstellen eines zahntechnischen Modells zu entnehmen. Dabei wird zunächst ein Kiefer gescannt, um sodann durch z. B. Rapid Prototyping ein Zahnteil herzustellen, das eine Basis mit einer oder mehreren Referenzierungen aufweist.

Der FR-A-2 715 826 ist ein Träger für ein zahntechnisches Modell zu entnehmen. Der Träger weist Durchgangsöffnungen auf, die bodenseitig von Stegen überbrückt sind.

Der vorliegenden Erfindung liegt die Aufgabe zugrunde, ein Verfahren, eine Tragplatte sowie eine zahntechnische Einrichtung der eingangs genannten Art so weiterzubilden, dass eine einfache Handhabbarkeit des zahntechnischen Modells sichergestellt ist, insbesondere ausgeschlossen ist, dass ein fehlerhaftes Einsetzen des zahntechnischen Modells bzw. der Segmente in die Halterung bzw. Tragplatte erfolgt. Gleichzeitig soll das zahntechnische Modell mit hoher Präzision hergestellt werden können, wobei gewünschte Materialien zum Einsatz gelangen sollen.

Die vorliegende Erfindung wird in Ansprüche 1, 7 und 13 definiert.

Nach einem weiteren Aspekt soll sichergestellt sein, dass die Segmente unabhängig von deren Breitenerstreckung in der Ausnehmung bzw. Aussparung der Halterung bzw. Tragplatte verkippungsfrei fixiert werden, wobei auch ein häufiges Entnehmen und Wiedereinsetzen zu keinen Unzulänglichkeiten führt.

Verfahrensmäßig wird die Aufgabe im Wesentlichen dadurch gelöst, dass das zahntechnische Modell durch unabhängig voneinander hergestellte jeweils zumindest ein Zahnteil und eine Basis aufweisende Segmente gebildet wird und dass jedes Segment als Einheit mit dem Zahnteil und der Basis unter Berücksichtigung der im Rechner abgespeicherten digitalisierten Daten des Kiefers oder eines Abschnitts von diesem und der im Rechner abgespeicherten Referenzierung der Ausnehmung hergestellt wird, wobei die Referenzierung ein positionsgenaues Einsetzen der Segmente in die Halterung sicherstellt. Bei der Referenzierung handelt es sich insbesondere um eine durch Vorsprünge bzw. Vertiefungen gebildete Struktur zumindest in einem Bereich der Ausnehmungen, in die die Basis eingesetzt wird, die mit dem zumindest einen Zahnteil als Einheit insbesondere im CAM (Computer Aided Manufacturing)-Verfahren hergestellt wird.

Abweichend vom vorbekannten Stand der Technik wird das zahntechnische Modell mit einer Basis hergestellt, die eine Referenzierung aufweist, die der das zahntechnische Modell aufnehmenden Halterung entspricht. Somit ist sichergestellt, dass nach der Herstellung des zahntechnischen Modells ein fehlerhaftes Einsetzen in die Halterung ausgeschlossen ist, insbesondere dann, wenn nur einzelne Segmente hergestellt werden, die sodann zum Herstellen des Zahnersatzes in der Halterung zum Modellieren des Gerüstes bzw. der Verblendung einzusetzen sind.

Die Basis und das Zahnteil sind als Einheit aus demselben Material wie z. B. Kunststoff hergestellt.

Ein zahntechnisches Modell umfasst üblicherweise ein Modell von Ober- und Unterkiefer. Daher sieht die Erfindung auch insbesondere vor, dass das zahntechnische Modell in Form von zumindest zwei aneinandergrenzenden Segmenten hergestellt wird, wobei zumindest ein Segment, vorzugsweise jedes Segment zumindest zwei Zahnteile mit gemeinsamer Basis aufweist.

Um zu verhindern, dass Segmente unkontrolliert verkippen können, wie dies insbesondere bei schmalen, z. B. nur einen Vorderzahn umfassenden Segmenten der Fall sein kann, sieht die Erfindung in einem selbständigen Lösungsvorschlag vor, dass die Aussparung unterseitig von einer Bodenwandung mit einem Längsschlitz begrenzt wird, wobei die Basis bodenseitig mit einem ersten Vorsprung zum Einsetzen in den Längsschlitz ausgebildet wird.

Durch den an den Längsschlitz angepassten Vorsprung ist ein Kippen des Segmentes ausgeschlossen.

Die sichere Fixierung wird insbesondere und gleichfalls nach einem selbständigen Lösungsvorschlag auch dann erzielt, wenn entsprechend der erfindungsgemäßen Lehre vorgesehen ist, dass das plattenförmige Element oberseitig einen zur Oberfläche zurückversetzten und entlang der Aussparung verlaufenden und in diese übergehenden ersten Abschnitt aufweist, und dass die Basis derart hergestellt wird, dass von dieser ein seitlich abragender zweiter Vorsprung ausgeht, der an den zurückversetzten ersten Abschnitt oder an einen Bereich von diesem angepasst wird.

Hierdurch liegt das Basisteil relativ großflächig auf der Halterung auf, wird folglich nicht - wie dies beim Stand der Technik der Fall ist - ausschließlich in der Aussparung selbst fixiert, sondern zusätzlich durch den seitlich abragenden zweiten Vorsprung, der auf einer parallel zur Oberfläche der Halterung verlaufenden Begrenzung des ersten Abschnitts aufliegt.

Bei dem zahntechnischen Modell selbst kann es sich um ein Meister- oder ein Arbeitsmodell handeln. Das Arbeitsmodell unterscheidet sich von dem Meistermodell dahingehend, dass bei ersterem an den Stümpfen, auf die der Zahnersatz aufzubringen ist, das Zahnfleisch entfernt ist. Dies ist wichtig, um die Präparationsgrenze bestimmen zu können. Es besteht die Möglichkeit, Segmente mit Präparationsgrenze und ohne Präparationsgrenze, also mit Zahnfleisch herzustellen. Im Falle, dass die Zahnfleischkontur nicht vorliegt, ergibt sich ein Segment mit Unterkehlung. Durch Austausch von identischen Segmenten, die somit identische Stümpfe aufweisen, ergibt sich der Vorteil, dass entweder ein Arbeits- oder Meistermodell zur Verfügung steht. Ein Arbeitsmodell liegt dann vor, wenn ein Segment ohne Gingiva eingesetzt ist. Ein Meistermodell steht dann zur Verfügung, wenn die Kontur der Gingiva vorliegt.

Die Segmente können auf Grund der vorliegenden digitalisierten Daten des intraoral gescannten Kiefers derart hergestellt werden, dass diese ohne Säge- bzw. Schnittspalt aneinanderreihbar sind. Nach dem Stand der Technik weist ein entsprechender Säge- bzw. Schnittspalt eine Breite zwischen 0,3 mm und 0,5 mm auf. Hierdurch gehen Informationen verloren. Erfindungsgemäß wird nur ein minimaler herstellungsbedingter Spalt benötigt, um das Einsetzen bzw. Herausnehmen der Segmente sicherzustellen. Die Breite des Spaltes beträgt weniger bzw. sehr viel weniger als 0,1 mm.

Eine Tragplatte der eingangs genannten Art zeichnet sich dadurch aus, dass die Oberseite der Tragplatte einen entlang der Aussparung verlaufenden zurückversetzten Bereich als zweite Führung für die Basis durch einen seitlich von der Basis abragenden an den zurückversetzten Bereich angepassten zweiten Vorsprung aufweist und die Aussparung bodenseitig einen entlang der Aussparung verlaufenden Schlitz als dritte Führung für die Basis durch Eingreifen eines von der Basis bodenseitig abragenden ersten Vorsprungs in den Schlitz aufweist.

Dabei ist insbesondere vorgesehen, dass sich die Aussparung mit dem Schlitz von der Oberseite bis zur Unterseite und diese durchsetzend erstreckt.

Hervorzuheben ist des Weiteren, dass der Schlitz unterseitig von zumindest einem quer zur Längsrichtung des Schlitzes verlaufenden Steg überbrückt sein kann. Dabei kann der den Schlitz überbrückende Steg, wobei vorzugsweise mehrere Stege vorgesehen sind, zusätzlich eine Führung bzw. eine Befestigung in einem Artikulator ermöglichen. Auf der Unterseite der Tragplatte können des Weiteren eine oder mehrere Aussparungen bzw. Vorsprünge vorhanden sein, um die Tragplatte eindeutig in einem Artikulator zu fixieren und zu befestigen. Hierzu kann - wie üblich - ein Magnet in zumindest eine Aussparung eingebracht werden.

Um zum einen das Einsetzen des zahntechnischen Modells bzw. Segmente von diesem zu erleichtern und zum anderen eine sichere Führung zu ermöglichen, sieht die Erfindung des Weiteren vor, dass die erste Führung durch die jeweils vorzugsweise eine Wellengeometrie aufweisenden Seitenflächen mit senkrecht zu der Oberseite der jeweiligen Seitenfläche verlaufenden Erhebungen und Vertiefungen gebildet ist, wobei insbesondere der Abstand der Erhebungen einer Seitenfläche vom Abstand der Erhebungen der anderen Seitenfläche abweicht, insbesondere der Abstand von zwei Erhebungen der entlang Außenbogen verlaufenden Seitenfläche 1,5- bis 10-fach, vorzugsweise 2- bis 5-fach so groß wie Abstand von zwei Erhebungen der gegenüberliegenden entlang Innenbogen verlaufenden Seitenfläche ist.

Selbstverständlich wird die Erfindung nicht verlassen, wenn nur eine der Seitenflächen, die die Referenzierung bilden, Erhebungen und Vertiefungen aufweist.

Eine zahntechnische Einrichtung der eingangs genannten Art ist dadurch gekennzeichnet, dass die Aussparung einen bodenseitig sich entlang der Aussparung erstreckenden Schlitz aufweist und die Basis einen an den Schlitz geometrisch angepassten, bodenseitig von der Basis abragenden ersten Vorsprung aufweist, und dass die Tragplatte oberseitig einen zu deren Oberfläche zurückversetzten entlang der Aussparung verlaufenden Bereich aufweist und die Basis einen geometrisch an den Bereich angepassten seitlich abragenden zweiten Vorsprung aufweist.

Vorzugsweise ist vorgesehen, dass zumindest eines der Segmente zumindest zwei Zahnteile aufweist.

Dabei ist insbesondere vorgesehen, dass die Segmente in der Aussparung aneinandergereiht angeordnet sind und dass zumindest zwei Zahnteile von einem gemeinsamen Basisabschnitt ausgehen.

Als bevorzugte Materialien für das zahntechnische Modell sind Kunststoffe, insbesondere gefüllte Kunststoffe, besonders bevorzugt gefüllte Polyurethan-Kunststoffe zu nennen.

Für die Halterung bzw. Tragplatte kommen Materialien wie Aluminium, Aluminiumlegierungen, Titan, Titanlegierungen, Stahl oder Kunststoffe in Frage, ohne dass hierdurch eine Einschränkung der erfindungsgemäßen Lehre erfolgen soll.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung eines den Zeichnungen zu entnehmenden bevorzugten Ausführungsbeispiels.

Es zeigen:
- Fig. 1: eine Tragplatte zur Aufnahme eines zahntechnischen Modells von oben,
- Fig. 2: eine der Fig. 1 entsprechende Tragplatte, betrachtet von unten, mit zusätzlichen Stegen,
- Fig. 3: ein in eine Tragplatte eingesetztes zahntechnisches Modell, die der Fig. 1 und 2 entspricht,
- Fig. 4: das zahntechnische Modell in der Tragplatte gemäß Fig. 3, wobei Segmente herausgezogen sind, die Stümpfe aufweisen,
- Fig. 5: die die Stümpfe aufweisenden Segmente, jedoch mit Unterkehlung,
- Fig. 6: eine zweite Ausführungsform einer Tragplatte,
- Fig. 7: ein Segment ohne Gingiva,
- Fig. 8: das Segment gemäß Fig. 7 mit Gingiva,
- Fig. 9: eine perspektivische Darstellung eines Segmentes, das in die Tragplatte gemäß Fig. 6 eingesetzt wird,
- Fig. 10: eine dritte Ausführungsform einer Tragplatte,
- Fig. 11: ein Segment, das in die Tragplatte gemäß Fig. 10 eingesetzt wird, und
- Fig. 12: eine vierte Ausführungsform einer Tragplatte

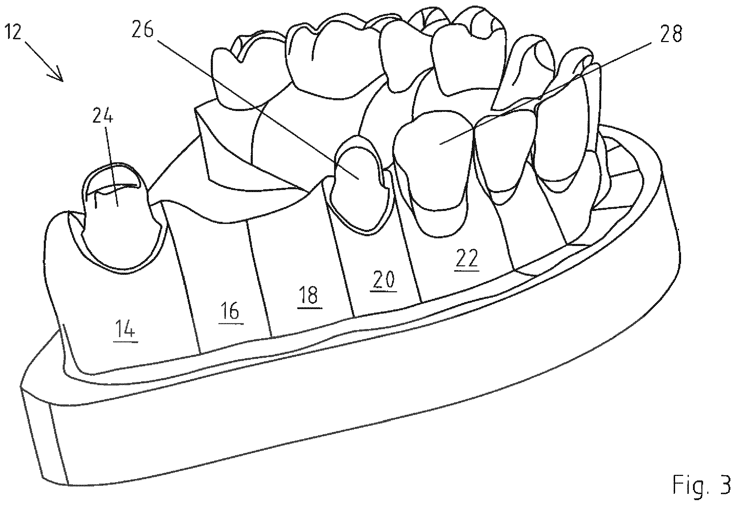

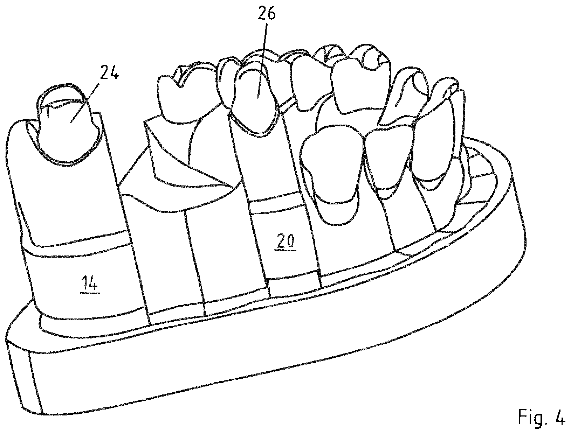

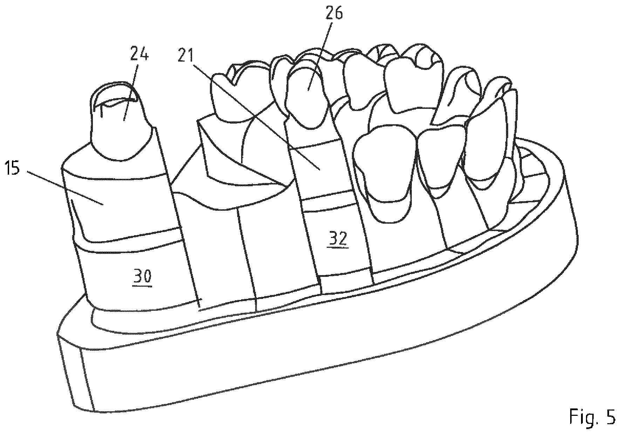

Den Zeichnungen sind Prinzipdarstellungen eines zahntechnischen Modells 12 zu entnehmen, das als Meistermodell (Fig. 3, Fig. 4) oder Arbeitsmodell (Fig. 5) ausgeführt sein kann und von einer nachstehend als Tragplatte 10, 100, 200, 300 bezeichneten Halterung aufgenommen wird. Dabei ist das zahntechnische Modell 12 in Segmente geschnitten bzw. unterteilt, von denen einige rein beispielhaft mit den Bezugszeichen 14, 16, 18, 20, 22 gekennzeichnet sind.

Die Segmente 14, 20, 22 weisen dabei Zahnteile 24, 26, 28 auf, von denen die Teile 24, 26 präpariert sind und als Stümpfe für eine aufzunehmende Brücke als Zahnersatz dienen.

Geht in der zeichnerischen Darstellung jedes Zahnteil von einem gesonderten Segment aus, so ist dies nicht zwingend. Insbesondere ist es auch nicht erforderlich, dass die Zahnteile, die nicht mit einem Zahnersatz zu versehen sind, segmentiert sind. Vielmehr können mehrere Zahnteile von einem einzigen Segment ausgehen.

Zur Herstellung des zahntechnischen Modells 12 wird intraoral der Kiefer bzw. Kieferbereich eines Patienten oder ein Abdruck des Kiefers bzw. Kieferbereichs gescannt, der mit einem Zahnersatz versorgt werden soll. Auf Grund der Scanndaten wird im CAM (Computer Aided Manufacturing)-Verfahren das zahntechnische Modell hergestellt, wobei der Teil, der über der Halterung 10 vorsteht, die Situation im Mund des Patienten wiedergibt. Die Scanndaten werden dabei in bekannter Weise registriert oder gematcht, da eine Vielzahl von Aufnahmen erforderlich ist, um den Kieferbereich umfassend und hinreichend genau zu scannen. Zusätzlich können fehlende oder nicht auswertbare Daten durch geeignete Software ergänzt werden. Insoweit wird jedoch auf hinreichend bekannte Techniken verwiesen.

Die Segmente 14, 16, 18, 20, 22 können einzeln und unabhängig voneinander nach einem optimierten Herstellungsverfahren produziert werden. Damit die Segmente 14, 16, 18, 20, 22 im gewünschten Umfang positionsgenau in die Halterung eingesetzt und nach einer Herausnahme auch wieder unter Beibehaltung der ursprünglichen Reihenfolge einfach zurückgesetzt werden können, ist erfindungsgemäß vorgesehen, dass die Zahnteile mit einer Basis 30, 32 hergestellt werden, die an eine in der Tragplatte 10, 100, 200, 300 vorhandene Referenzierung angepasst ist, wodurch das positionsgenaue Einsetzen der Segmente 14, 16, 18, 20, 22 in die Tragplatte 10, 100 gewährleistet ist. Das bzw. die Zahnteil(e) mit der Basis werden als Einheit insbesondere aus Kunststoff hergestellt.

Wird von Basisteil eines Zahnteils gesprochen, so gilt dies auch für den Kieferbereich, wo sich kein Zahn mehr befindet, - wie bei den Segmenten 16, 18 -. Insoweit ist Zahnteil umfassend zu verstehen und schließt auch Bereiche ein, in denen sich ausschließlich der Kieferknochenbereich mit der Gingiva erstreckt. Der Begriff Zahnteil ist umfassend zu verstehen und schließt auch künstliche Zahnstümpfe, Implantatpfosten, Wurzelaufbauten oder ähnliches ein.

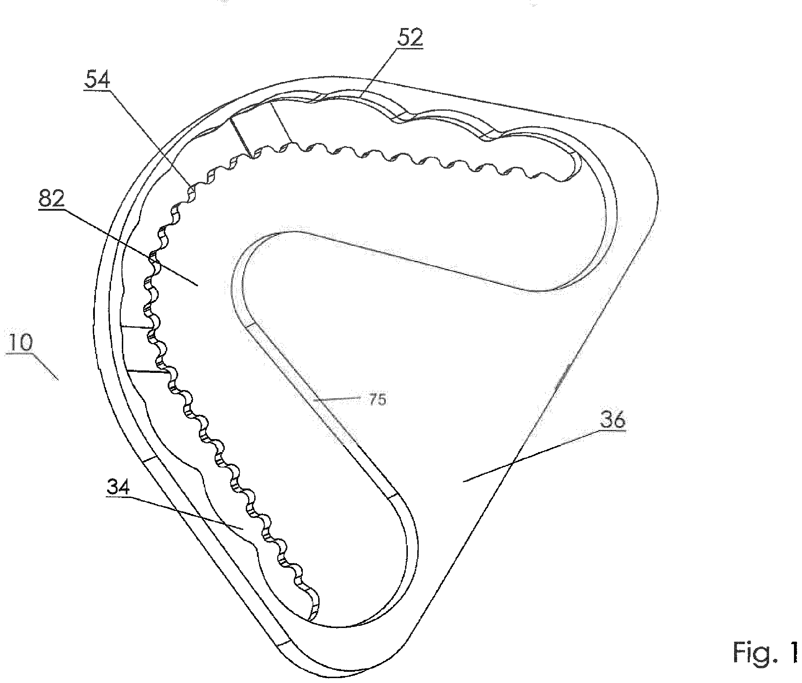

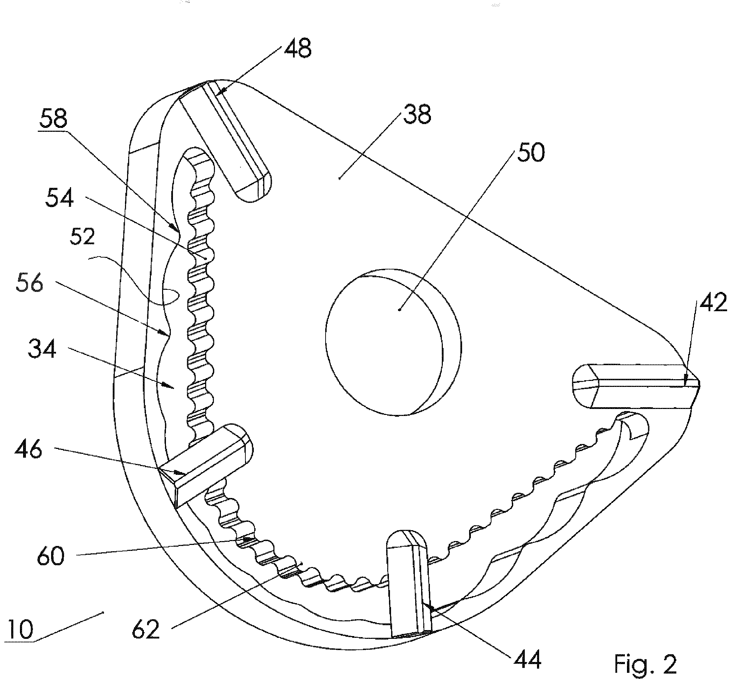

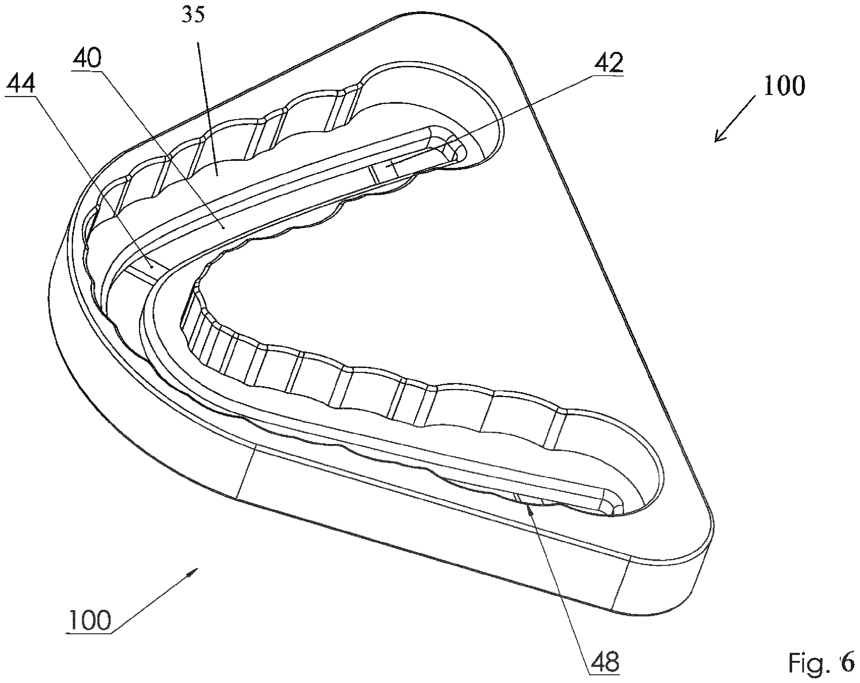

Wie sich aus den Fig. 1 und 2 ergibt, weist die Tragplatte 10, 100 zur Aufnahme des zahntechnischen Modells 12 eine in etwa dem Verlauf eines Kieferbogens aufweisende durchgehende Aussparung 34 - auch Ausnehmung genannt - auf, die von der Oberseite 36 der Tragplatte ausgehend sich bis zur Unterseite 38 erstreckt. Verläuft bei dem Ausführungsbeispiel der Fig. 1 und 2 die Aussparung 34 vollständig von der Oberseite 36 bis zur Unterseite 38 und diese durchsetzend, so ist nach dem Ausführungsbeispiel der Fig. 6 die Aussparung 34 von einer Bodenwandung 35 begrenzt, die ihrerseits von einem Schlitz 40 durchsetzt ist. Selbstverständlich kann die Bodenwandung 35 auch geschlossen sein.

Entsprechend der Darstellung der Fig. 1, 2 und 6 erstreckt sich entlang der Unterseite 38 der Tragplatte 10, 100 die Aussparung 34 bzw. den Schlitz 40 überbrückend Stege 42, 44, 46, 48, um eine hinreichende Formstabilität der Tragplatte 10, 100 sicherzustellen.

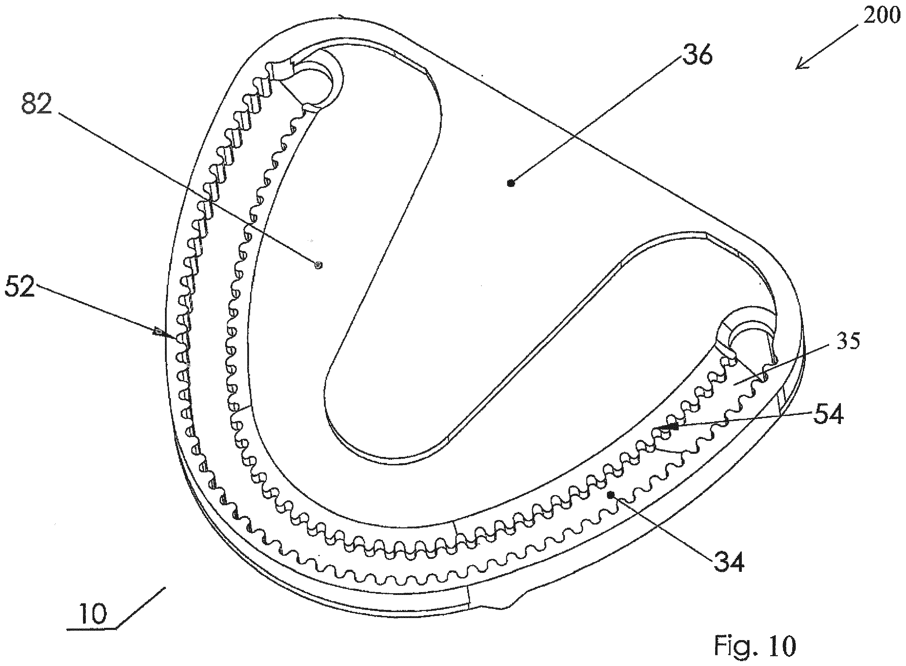

In Fig. 10 ist eine Tragplatte 200 dargestellt, in der die Aussparung 34 von einer geschlossenen Bodenwandung 35 begrenzt ist.

Die Stege 42, 44, 46, 48 können gleichfalls als Fixierung und Montagehilfe für die Tragplatte 10, 100 auf einem Artikulator dienen. Zusätzlich kann eine zylindrische Aussparung 50 in der Bodenfläche, also Unterseite 38 der Tragplatte 10 vorgesehen sein, um die Tragplatte 10 in einem Artikulator befestigen zu können, z. B. mit einem Magneten.

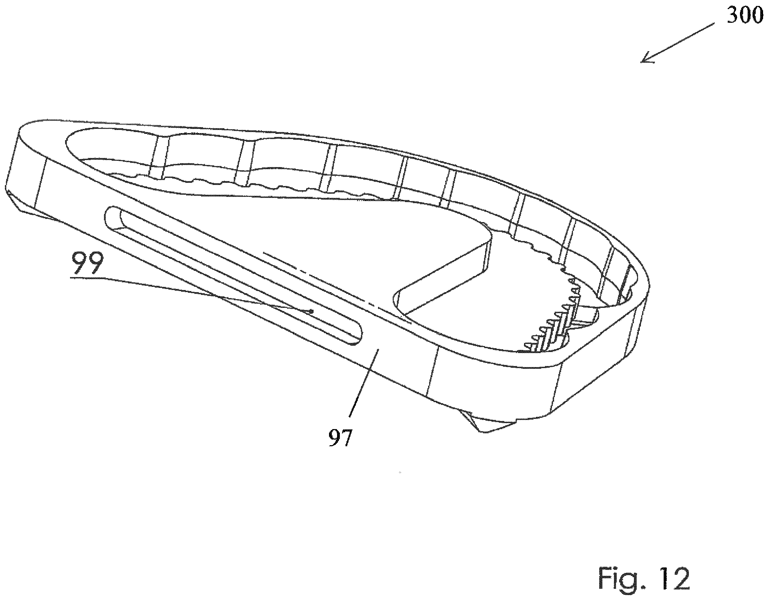

Eine in der Fig. 12 dargestellte Tragplatte 300, die prinzipiell der Tragplatte 10 gemäß den Fig. 1 und 2 entspricht, weist zur Fixierung in einem Artikulator in ihrer Basisseitenfläche 97 einen Längsschlitz 99 auf. Die Seitenfläche 97 ist als Basisseitenfläche bezeichnet, da der Träger 300 in Draufsicht in etwa eine Dreieckform mit abgerundeten Ecken aufweist, wobei die den Längsschlitz 99 aufweisende Seitenfläche 97 die Basis eines entsprechenden Dreiecks ist.

Die Referenzierung der Aussparung 34, um das zahntechnische Modell 12, also dessen Basis bzw. Abschnitten von diesen bzw. dessen Basisteile 30, 32, die insgesamt die Basis bilden, positionsgenau einsetzen zu können, wird dadurch gebildet, dass die Seitenflächen 52, 54 strukturiert sind, und zwar im Ausführungsbeispiel durch eine wellen- oder zahnförmige Geometrie, ohne dass hierdurch eine Einschränkung der erfindungsgemäßen Lehre erfolgen soll. Andere geeignete mechanische Referenzierungen sind gleichfalls möglich, durch die sichergestellt wird, dass die Segmente 14, 16, 18, 20, 22 in einer einzigen Position und damit eindeutig in die Aussparung 34 einsetzbar sind.

Entsprechend der zeichnerischen Darstellungen der Fig. 1 und 2 unterscheiden sich die Begrenzungen der Seitenwandungen 52, 54 dadurch, dass der Abstand der Erhebungen 56, 58 der entlang des Außenbogens verlaufenden Seitenfläche 52 der Ausnehmung oder Aussparung 34 größer als der Abstand der Erhebungen 60, 62 der innen verlaufenden Seitenfläche 54 des Innenbogens ist, wobei insbesondere der Abstand zwischen zwei bis vier Erhebungen 60, 62 der Innenfläche 54 gleich dem Abstand von zwei aufeinanderfolgenden Erhebungen 56, 58 der äußeren Innenfläche 52 sein sollte.

Bei dem Ausführungsbeispiel der Fig. 10 weist die Tragplatte 200 in ihren Seitenwandungen 52, 54 jeweils eine Wellenstruktur auf, die in Bezug auf den Abstand der Erhebungen in etwa gleich ist, ohne dass die erforderliche Individualisierung verlorengeht, die erforderlich ist, damit die Zahnteile in einer einzigen eindeutigen Position in die Ausnehmung 34 und damit die Traplatte 200 einsetzbar sind.

Bieten die Strukturen, also der wellenförmige Verlauf der Seitenbegrenzungen, also der Innenflächen 52, 54 der Ausnehmung oder Aussparung 34 die Möglichkeit einer eindeutigen Positionierung der Segmente 14, 16, 18, 20, 22, indem die Basisteile 30, 32 entsprechend strukturiert sind, so wird durch den Längsschlitz 40 sichergestellt, dass ein ungewünschtes Kippen der Segmente, insbesondere im Vorderzahnbereich unterbleibt, also in dem Bereich des Kiefers, wo die kleinste Passfläche bei größter Krümmung vorliegt. Hierzu weisen die Basisteile an die Geometrie des Längsschlitzes 40 angepasste Vorsprünge - auch erste Vorsprünge genannt - auf, die in der Fig. 6 angedeutet und mit dem Bezugszeichen 64 gekennzeichnet sind.

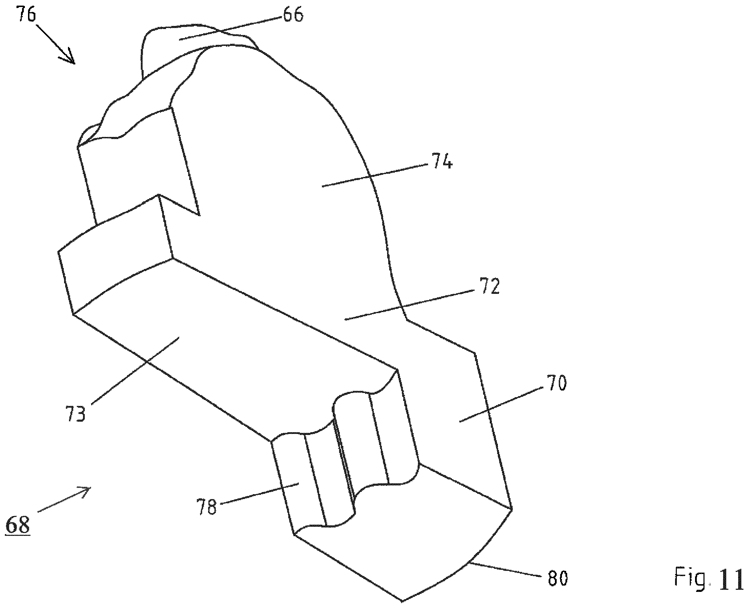

Anhand der Fig. 11 wird deutlich, dass eine ein Zahnteil 66 aufweisende Basis 68, auch Basisteil genannt, aus einem unteren Abschnitt 70, einem mittleren Abschnitt 72 und einem oberen Abschnitt 74 bestehen kann, der in den Kieferbereich mit dem Zahn 66 übergeht, wobei dessen Geometrie bzw. deren Geometrien aus den durch Scannen gewonnenen digitalisierten Daten ermittelt werden.

Der untere Abschnitt 70 des Basisteils 68 wird in die Ausnehmung 34 eingesetzt. Man erkennt, dass die in den Darstellungen der Fig. 11 vordere Fläche des unteren Abschnitts 70 des Basisteils 68 eine Wellengeometrie aufweist, die der der inneren Innenfläche 54 der Aussparung 34 der Fig. 1, 2 entspricht. Demgegenüber ist die hintere Fläche des unteren Abschnitts 70 des Basisteils 68 ebener ausgebildet, da die Wellengeometrie der äußeren Innenfläche 52 auseinandergezogen ist, wie die Fig. 1, 2 verdeutlichen.

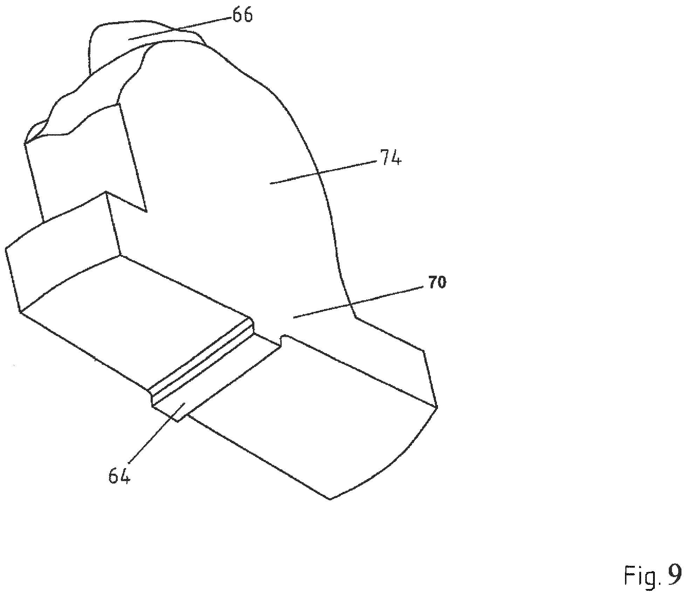

Der mittlere Abschnitt 72 liegt auf der Oberseite 36 der Tragplatte 10 auf, und zwar in einem zurückversetzten Bereich 82, wie sich aus den Fig. 1, 12 ergibt. Der zurückversetzte Bereich 82 wird als erster Abschnitt bezeichnet. Somit ergibt sich ein großflächiges Anliegen des Basisteils 68 auf der Tragplatte 10, 300, wodurch zusätzlich ein Verkippen des Segments unterbunden wird, und zwar in einem Umfang, dass gegebenenfalls der Schlitz 40 und der an diesen angepasste Vorsprung 64 (Fig. 9) an der Unterseite des unteren Abschnitts des Basisteils 68 entfallen kann.

Unabhängig hiervon sollte der Schlitz 40 mittig zwischen den Seitenflächen 52, 54 verlaufen. Der Schlitz 40 weist dann, wenn er die Bodenwandung 35 durchsetzt, parallel zueinander verlaufende Wandungen auf. Ist der Schlitz 40 quasi als Nut ausgebildet, so sollte dieser im Schnitt eine U- oder V-Form aufweisen.

Des Weiteren ist zu den Seiten- oder Innenflächen 52, 54 anzumerken, dass diese senkrecht zu der Ober- und Unterseite 36, 38 verlaufen.

Sowohl die durch die Strukturierung der Innen- oder Seitenflächen 52, 54 der Ausnehmung oder Aussparung 34 vorgegebene Referenzierung als auch die Geometrie des zurückversetzten Bereichs 82 in der Oberseite 36 der Tragplatte 10 und - sofern vorhanden - der Verlauf und die Geometrie des Schlitzes 62 sind datenmäßig in einem Rechner abgelegt, in dem auch die durch intraorales Scannen oder Scannen eines Abdrucks gewonnenen Zahn- und Kieferdaten abgespeichert werden. Die entsprechenden Daten werden sodann verknüpft, um einer Bearbeitungsmaschine zugeführt zu werden, um das zahntechnische Modell 12, d.h. dessen Segmente 14, 16, 18, 20, 22 zu produzieren. Übliche Bearbeitungen z.B. mittels Drehfräsens eines Rohlings werden mittels der Bearbeitungsmaschine durchgeführt, um die Segmente 14, 16, 18, 20, 22 herzustellen. Dabei können diese eine derartige Geometrie aufweisen, dass sich zwischen den Segmenten 14, 16, 18, 20, 22 ein Schnitt- bzw. Sägespalt nicht ergibt, wie dieser bei ansonsten hergestellten Modellen auftreten muss. Auch kann gezielt ein Pseudo-Sägeschnitt erzeugt werden.

Unabhängig hiervon sollten die Segmente 14 - 22 dann, wenn diese in der Aussparung 34 eingesetzt sind, einen Abstand A von weniger bzw. viel weniger als 0,1 mm aufweisen.

Zu erwähnen ist, dass die Unterseite 73 des mittleren Abschnitts 72 nicht nur auf der Fläche des zurückversetzten Abschnitts 82 der Tragplatte 10 aufliegt, sondern dass äußere freie Begrenzungsfläche 74, die sich senkrecht zu der Unterseite 73 des mittleren Abschnitts 72 verläuft, an der eine Stufe zu der Oberseite 36 bildenden Wandung 75 des zurückversetzten Bereichs 82 anliegt.

Der zurückversetzte Abschnitt oder Bereich 82 bildet nicht nur eine Führung für die Basis bzw. einen Abschnitt der Basis eines Segments, sondern erleichtert auch das Herausnehmen des Segments aus der Aussparung 34. Durch die relativ große im Wesentlichen senkrecht zur Aussparung verlaufende Erstreckung des mittleren Abschnitts 72 kann dann, wenn auf das obere Teil 74 eine Kraft eingeleitet wird, eine Hebelwirkung aufgrund der Erstreckung des mittleren Abschnitts 72 genutzt werden, wodurch der passgenau in die Aussparung 34 eingesetzte untere Abschnitt 70 der Basis 68 aus der Aussparung 34 gelöst werden kann.

Das der Fig. 9 zu entnehmende Segment wird in die Tragplatte bzw. Halterung 100 gemäß Fig. 6 eingesetzt. Man erkennt, dass von der Unterseite der Basis bzw. dessen unterem Abschnitt 70, der in die Ausnehmung 34 eingesetzt wird, der Steg 64 abragt, der an die Geometrie des Schlitzes 40 in der Tragplatte 100 angepasst ist.

Ein Vergleich der Fig. 3 und 4 zeigt eine Situation, in der die Segmente 14 und 20, die die Stümpfe 24, 26 aufweisen, aus der Ausnehmung 34 der Tragplatte 10 entfernt werden bzw. in diese eingesetzt werden sollen. Dabei bilden die Segmente 14, 20 mit den weiteren Segmenten ein Meistermodell, bei dem die tatsächliche Kontur der Gingiva vorhanden ist.

Um jedoch die Präparationsgrenze zu ermitteln, entlang der der nicht dargestellte Zahnersatz verläuft, besteht gleichfalls auf Grund des Scannens die Möglichkeit, die Stümpfe 24, 26 ohne Gingiva herzustellen, so dass sich bei Verwendung entsprechender Segmente 15, 21, die in Bezug auf ihre Basisteile 30, 32 identisch mit den Segmenten 14, 20 übereinstimmen, ein Arbeitsmodell mit unterhalb der Zahnstümpfe 24, 26 vorhandenen Unterkehlungen ergibt, durch die die Präparationsgrenze ersichtlich ist.

Auf einem entsprechenden Arbeitsmodell wird sodann die Zahnkonstruktion durch manuelles Formen oder durch direktes Verwenden der Scanndaten zum Herstellen eines Gerüstes durch ein CAD/CAM-Verfahren hergestellt. Letztere Möglichkeit ist zu bevorzugen. Anschließend wird das entsprechende Gerüst auf die Zahnstümpfe 24, 26 aufgesetzt, wobei allerdings die Segmente 15, 21 durch die Segmente 14, 20 ersetzt sind, die die Gingiva aufweisen. Es wird folglich erneut das Meistermodell benutzt. Dies ist notwendig, um festzustellen, ob die Rekonstruktion passgenau die Zahnstümpfe 24, 26 bis zur Gingiva umgeben. Anschließend erfolgt in üblicher Technik ein Verblenden der Gerüste.

Unabhängig hiervon ist anzumerken, dass der Begriff Gerüst ganz allgemein zu verstehen ist, also nicht nur Brückengerüste, sondern auch Kronen, Onlays, Inlays oder andere Zahnrekonstruktionen einschließt. Insoweit ist der Begriff Gerüst als Synonym zu verstehen.

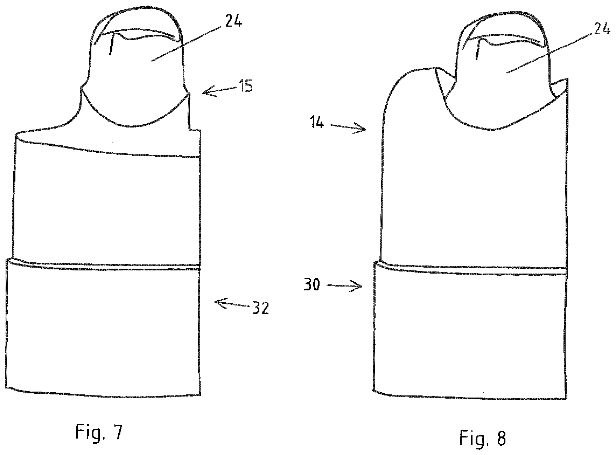

In den Fig. 7 und 8 sollen noch einmal rein prinzipiell die Segmente 14 und 15 dargestellt werden. Man erkennt, dass das Segment 14 in Bezug auf seinen Zahnstumpf 24 von der Gingiva umgeben ist, wohingegen der Zahnstumpf 24 des Segments 15 eine Unterkehlung aufweist, also die Gingiva entfernt worden ist.

Die Basisteile 30, 32 der Segmente 14, 15 stimmen identisch überein, so dass eine Platzierung der Segmente 14, 15 nur an ein und demselben Ort einer Tragplatte erfolgen kann. Dies wird durch die Referenzierung der entsprechenden Aussparung 34 in der Tragplatte 10, 100, 200, 300 sichergestellt, die entsprechend der zu einzunehmenden Positionen der Segmente 14, 15 in den Basisabschnitten 30, 32 abgebildet ist.

Auf Grund der erfindungsgemäßen Lehre wird erstmalig ein zahntechnisches Modell zur Verfügung gestellt, bei dem bei der Herstellung automatisch ein Basisteil mit ausgebildet wird, dass eine Referenzierung aufweist, die der einer Tragplatte - die auch als Halteplatte oder Halterung bezeichnet werden kann - entspricht, so dass eine eindeutige Positionierung sichergestellt ist. Infolgedessen kann der Nutzer ohne weitere Anleitung die Segmente einsetzen, da für jedes Segment nur eine einzige Positioniermöglichkeit gegeben ist.

Bezüglich der Referenzierung ist anzumerken, dass auch andere Geometrien als die beschriebene Wellengeometrie in Frage kommen. Dem Grunde nach kommen beliebige Strukturen in Frage, sofern keine abrupten Richtungswechsel wie z. B. Zacken vorhanden sind, die eine Herstellung mit einem abtragenden Verfahren wie Fräsen unnötig erschweren würden.

Vorteile der Erfindung ergeben sich auch aus Folgendem. So kann ein Zahnarzt einen Kiefer scannen, um sodann auf der Basis dieser Daten einzelne Segmente herstellen zu lassen. Die z. B. fremdgefertigten Segmente werden sodann dem Zahnarzt bzw. einem Zahntechniker zur Verfügung gestellt, der problemlos die Segmente in eine ihm vorliegende Halterung einsetzen kann, und zwar auf Grund der erfindungsgemäßen Ausbildung der Basis mit der vorgegebenen Referenzierung, die die Halterung in ihrer Ausnehmung aufweist. Zusätzlich können die Segmente eine optisch erfassbare Referenzierung wie Nummerierung aufweisen, um ein Probieren des Einsetzens in die Ausnehmung zu vermeiden. Durch eine entsprechende optische Markierung wie Nummerierung wird die grobe Ausrichtung des Segmentes auf die Aussparung in der Halterung erleichtert. Das präzise Einsetzen wird durch die Referenzierung - also mechanische Struktur - an der Basis und der zugeordneten Referenzierung der Aussparung in der Halterung sichergestellt.

## Patentansprüche

1. Verfahren zum Herstellen eines in eine eine Referenzierung aufweisende Aussparung bzw. Ausnehmung (34) einer Halterung (10, 100, 200, 300) lösbar einzusetzenden zahntechnischen Modells (12) umfassend zumindest ein Zahnteil (24, 26, 28, 66) mit einer Basis (30, 32, 68), die oder ein Abschnitt von dieser in die Ausnehmung eingesetzt wird, wobei unter Verwendung von digitalisierten Daten eines Kiefers oder eines Abschnitts von diesem das zumindest eine Zahnteil hergestellt wird, indem zuvor die digitalisierten Daten in einem Rechner abgespeichert und unter Verwendung dieser einer Bearbeitungsmaschine zur Herstellung des zumindest einen Zahnteils zugeführt werden,
**dadurch gekennzeichnet,**
**dass** das zahntechnische Modell (12) durch unabhängig voneinander hergestellte jeweils zumindest ein Zahnteil (24, 26, 28, 66) und eine Basis (30, 32, 38) aufweisende Segmente (14, 16, 18, 20, 22) gebildet wird und dass jedes Segment als Einheit mit dem Zahnteil und der Basis unter Berücksichtigung der im Rechner abgespeicherten digitalisierten Daten des Kiefers oder eines Abschnitts von diesem und der im Rechner abgespeicherten Referenzierung der Ausnehmung hergestellt wird, wobei die Referenzierung ein positionsgenaues Einsetzen der Segmente in die Halterung sicherstellt.

2. Verfahren nach Anspruch 1,
**dadurch gekennzeichnet,**
**dass** das zahntechnische Modell in Form von zumindest zwei aneinander grenzenden Segmenten (14, 16, 18, 20, 22, 24) hergestellt wird, wobei zumindest ein Segment, vorzugsweise jedes Segment zumindest zwei Zahnteile mit gemeinsamer Basis (30, 32, 68) aufweist.

3. Verfahren nach Anspruch 1 oder 2,
**dadurch gekennzeichnet,**
**dass** als Halterung (10, 100, 200, 300) ein plattenförmiges Element mit Ober- und Unterseite (36, 38) und die einen bogenförmigen Verlauf aufweisende Aussparung bzw. Ausnehmung (34) verwendet wird, die von Seitenflächen (52, 54) begrenzt ist, die mit der Referenzierung versehen ist.

4. Verfahren nach zumindest einem der vorhergehenden Ansprüche,
**dadurch gekennzeichnet,**
**dass** die Aussparung (34) unterseitig von einer Bodenwandung mit in dieser vorhandenem Längsschlitz (40) begrenzt wird, der gegebenenfalls die Bodenwandung durchsetzt, wobei die Basis (30, 32, 68) bodenseitig mit einem ersten Vorsprung (64) zum Einsetzen in den Längsschlitz ausgebildet wird.

5. Verfahren nach zumindest einem der vorhergehenden Ansprüche,
**dadurch gekennzeichnet,**
**dass** das plattenförmige Element oberseitig einen zur Oberfläche zurückversetzten und entlang der Ausnehmung (34) verlaufenden und in diese übergehenden ersten Abschnitt (82) aufweist, und dass die Basis (64) derart hergestellt wird, dass von dieser ein seitlich abragender zweiter Vorsprung (72) ausgeht, der an den zurückversetzten ersten Abschnitt oder an einen Bereich von diesem angepasst wird.

6. Verfahren nach zumindest einem der vorhergehenden Ansprüche,
**dadurch gekennzeichnet,**
**dass** auf der Grundlage des zahntechnischen Modells ein Zahnersatz manuell und/oder mittels CAD/CAM-Verfahren hergestellt wird.

7. Tragplatte (10, 100) zur Aufnahme von einem aus mehreren Segmenten bestehenden Gebissmodell (12), wobei jedes Segment eine Basis (68) mit einem Zahnteil (66) oder mehreren Zahnteilen aufweist, und wobei die Tragplatte eine Ober- und Unterseite (36, 38) und eine einem Bogen wie Gebissbogen folgende Ausnehmung bzw. Aussparung (34) mit Seitenflächen (52, 54) aufweist, die bzw. von denen zumindest eine der Seitenflächen eine mechanische Referenzierung aufweisen bzw. aufweist, die eine erste Führung für die Basis bildet, wobei die Basis und das Zahnteil als Einheit aus demselben Material hergestellt sind,
**dadurch gekennzeichnet,**
**dass** die Oberseite (36) der Tragplatte (10, 100) einen entlang der Aussparung (34) verlaufenden zurückversetzten Bereich (82) als zweite Führung für die Basis (68) durch einen seitlich von der Basis abragenden an den zurückversetzten Bereich angepassten zweiten Vorsprung (72) aufweist und die Aussparung bodenseitig einen entlang der Aussparung verlaufenden Schlitz (40) als dritte Führung für die Basis durch Eingreifen eines von der Basis bodenseitig abragenden ersten Vorsprungs (64) in den Schlitz aufweist.

8. Tragplatte nach Anspruch 7,
**dadurch gekennzeichnet,**
**dass** der Schlitz (40) als eine im Schnitt vorzugsweise V- oder U-förmige Aussparung in der Bodenwandung (35) ausgebildet ist oder die Bodenwandung durchsetzt.

9. Tragplatte nach Anspruch 7 oder 8,
**dadurch gekennzeichnet,**
**dass** der Schlitz (40) unterseitig von zumindest einem quer zur Längsrichtung des Schlitzes verlaufenden Steg (42, 44, 46, 48) überbrückt ist.

10. Tragplatte nach zumindest einem der Ansprüche 7 bis 9,
**dadurch gekennzeichnet,**
**dass** die erste Führung durch die jeweils eine Wellengeometrie aufweisenden Seitenflächen (52, 54) mit senkrecht zu der Oberseite (36) verlaufenden Erhebungen (56, 58, 60, 62) und Vertiefungen gebildet ist.

11. Tragplatte nach zumindest einem der Ansprüche 7 bis 10,
**dadurch gekennzeichnet,**
**dass** Abstand der Erhebungen (56, 58) einer Seitenfläche (52) vom Abstand der Erhebungen (60, 62) der anderen Seitenfläche (54) abweicht, insbesondere der Abstand von zwei Erhebungen der entlang Außenbogens verlaufenden Seitenfläche 1,5 bis 10-fach, vorzugsweise 2- bis 5-fach so groß wie Abstand von zwei Erhebungen der gegenüberliegenden entlang Innenbogen verlaufenden Seitenfläche ist.

12. Tragplatte nach zumindest einem der Ansprüche 7 bis 11,
**dadurch gekennzeichnet,**
**dass** der zumindest eine Steg (42, 44, 46, 48) Führung und/oder Befestigung für einen Artikulator ist.

13. Zahntechnische Einrichtung umfassend ein zahntechnisches Modell (12) bestehend aus zumindest zwei Segmenten (14, 16, 18, 20, 22) sowie eine das zahntechnische Modell lösbar in einer Ausnehmung bzw. Aussparung (34) aufnehmende Tragplatte (10, 100), wobei jedes Segment zumindest ein von einer Basis (68) ausgehendes Zahnteil (66), vorzugsweise zumindest zwei Zahnteile, aufweist, und das Zahnteil und die Basis als Einheit aus demselben Material hergestellt sind,
**dadurch gekennzeichnet,**
**dass** die Aussparung (34) einen bodenseitig sich entlang der Aussparung erstreckenden Schlitz (40) aufweist und die Basis einen an den Schlitz geometrisch angepassten, bodenseitig von der Basis abragenden ersten Vorsprung (64) aufweist, dass die Tragplatte (10, 100) oberseitig einen zu deren Oberfläche zurückversetzten entlang der Aussparung (34) verlaufenden Bereich (82) aufweist und die Basis (68) einen geometrisch an den Bereich angepassten seitlich abragenden zweiten Vorsprung (72) aufweist.

14. Zahntechnische Einrichtung nach Anspruch 13,
**dadurch gekennzeichnet,**
**dass** die Segmente (14, 16, 18, 20, 22) in der Aussparung (34) aneinandergereiht angeordnet sind und dass vorzugsweise zumindest zwei Zahnteile von einer gemeinsamen Basis ausgehen.

15. Zahntechnische Einrichtung nach Anspruch 13,
**dadurch gekennzeichnet,**
**dass** die in die Tragplatte (10, 100) eingesetzten vorzugsweise im CAM-Verfahren hergestellten Segmente (14, 16, 18, 20, 22) einen Abstand A mit A < 0,1 mm, insbesondere A << 0,1 mm aufweisen.

## Claims

1. Method for manufacturing a dental working model (12) to be detachably inserted into a cutout or recess (34) of a holder (10, 100, 200, 300) having referencing, comprising at least one tooth part (24, 26, 28, 66) with a base (30, 32, 68), which or of which a section is inserted into said recess, the at least one tooth part being manufactured using digitized data of a jaw or a section thereof in that the digitized data are stored beforehand in a computer and with the use thereof are supplied to a machine tool for manufacturing the at least one tooth part,
**characterized in**
**that** the dental working model (12) is formed by segments (14, 16, 18, 20, 22) manufactured independently of one another and each having at least one tooth part (24, 26, 28, 66) and one base (30, 32, 38), and each segment is manufactured as a unit with the tooth part and the base, taking into account the digitized data of the jaw or of a section thereof stored in the computer and the referencing of the recess stored in the computer, said referencing ensuring a precisely positioned insertion of the segments into the holder.

2. Method according to Claim 1,
**characterized in**
**that** the dental model is produced in the form of at least two mutually adjoining segments (14, 16, 18, 20, 22, 24), wherein at least one segment, preferably each segment, has at least two tooth parts with common base (30, 32, 68).

3. Method according to Claim 1 or 2,
**characterized in**
**that**, as holder (10, 100, 200, 300), a plate-shaped element having an upper and lower face (36, 38) as well as the recess or cut-out (34) having a curved shape is used, which is delimited by side surfaces (52, 54), which is provided with the referencing.

4. Method according to at least one of the previous claims,
**characterized in**
**that** the cut-out (34), on the underside, is delimited by a bottom wall with longitudinal slit (40) present in said bottom wall, longitudinal slit which optionally passes continuously through the bottom wall, wherein the base (30, 32, 68), on the underside, is formed with a first projection (64) for the insertion in the longitudinal slit.

5. Method according to at least one of the previous claims,
**characterized in**
**that** the plate-shaped element, on the upper face, has a first section (82) which is set back relative to the surface and which extends along the recess (34) and transitions into said recess, and in that the base (64) is produced in such a manner that, from said base, a laterally protruding second projection (72) originates, which is adapted to the set back first section or to an area thereof.

6. Method according to at least one of the previous claims,
**characterized in**
**that**, on the basis of the dental model, a dental prosthesis is manufactured manually and/or by CAD/CAM methods.

7. Carrying plate (10, 100) for receiving a denture model (12) consisting of several segments, each segment having a base (68) with one tooth part (66) or several tooth parts, and the supporting frame having an upper side and a lower side (36, 38) and a recess or cutout (34) following an arch such as a denture arch with lateral surfaces (52, 54), which or of which at least one of said lateral surfaces has or have mechanical referencing which forms a first guide for the base, said base and the tooth part being manufactured as a unit from the same material,
**characterized in**
**that** the upper side (36) of the carrying plate (10, 100) has a set-back area (82) along the cutout (34) as a second guide for the base (68) by a second projection (72) protruding laterally from the base and adapted to said set-back area, and the cutout has on the bottom side a slot (40) along the cutout as a third guide for the base by engagement in the slot of a first projection (64) protruding from the base on the bottom side.

8. Carrying plate according to Claim 7,
**characterized in**
**that** the slit (40) is formed as a cut-out with preferably a V- or U-shaped cross section in the bottom wall (35) or passing continuously through the bottom wall.

9. Carrying plate according to Claim 7 or 8,
**characterized in**
**that** the slit (40) is bridged on the underside by at least one rib (42, 44, 46, 48) which extends transversely to the longitudinal direction of the slit.

10. Carrying plate according to at least one of Claims 7 to 9,
**characterized in**
**that** the first guide is formed by the side surfaces (52, 54) each having a wave geometry, with bumps (56, 58, 60, 62) and indentations extending perpendicularly to the upper face (36).

11. Carrying plate according to at least one of Claims 7 to 10,
**characterized in**
**that** the distance between the bumps (56, 58) of one side surface (52) differs from the distance between the bumps (60, 62) of the other side surface (54), in particular the distance between two bumps of the side surface extending along the outer arch is 1.5 to 10 times, preferably 2 to 5 times greater than the distance between two bumps of the opposite side surface extending along inner arcs.

12. Carrying plate according to at least one of Claims 7 to 11,
**characterized in**
**that** the at least one rib (42, 44, 46, 48) is guide and/or fastening for an articulator.

13. Dental device comprising a dental working model (12) consisting of at least two segments (14, 16, 18, 20, 22) and a carrying plate (10, 100) detachably holding the dental working model inside a recess or cutout (34), each segment having at least one tooth part (66) extending from a base (68), and preferably at least two tooth parts, and said tooth part and said base being manufactured as a unit from the same material,
**characterized in**
**that** the cutout (34) has a slot (40) extending along the cutout on the bottom side and the base has a first projection (64) geometrically adapted to the slot and protruding from the base on the bottom side, the carrying plate (10, 100) has on the upper side an area (82) set back from its surface along the cutout (34), and the base (68) has a second projection (72) protruding laterally and geometrically adapted to said area.

14. Dental device according to Claim 13,
**characterized in**
**that** the segments (14, 16, 18, 20, 22) are arranged one after the other in the cut-out (34), and in that preferably at least two tooth parts originate from a common base.

15. Dental device according to Claim 13,
**characterized in**
**that** the segments (14, 16, 18, 20, 22) inserted in the carrying plate (10, 100) have a gap A where A < 0.1 mm, in particular A << 0.1 mm, said segments (14, 16, 18, 20, 22) are produced preferally by the CAM method.

## Revendications

1. Procédé de fabrication d'un modèle de travail dentaire (12) à insérer de manière amovible dans une cavité ou un évidement (34) présentant un référencement d'un support (10, 100, 200, 300) qui comprend au moins une partie de dent (24, 26, 28, 66) avec une base (30, 32, 68), ladite base ou une section de cette dernière étant insérée dans l'évidement, sachant qu'au moins une partie de dent est fabriquée en utilisant des données numérisées d'une mâchoire ou d'une section de celle-ci, en enregistrant au préalable les données numérisées dans un ordinateur et en les transmettant à une machine d'usinage pour produire au moins une partie de dent,
**caractérisé en ce**
**que** le modèle de travail dentaire (12) est formé par des segments (14, 16, 18, 20, 22) fabriqués indépendamment les uns des autres, comprenant chacun au moins une partie de dent (24, 26, 28, 66) et une base (30, 32, 38) et que chaque segment est fabriqué sous forme d'unité avec la partie de dent et la base, en tenant compte des données numérisées de la mâchoire ou d'une section de cette dernière enregistrées dans l'ordinateur et du référencement de l'évidement mémorisé dans l'ordinateur, sachant que le référencement assure une insertion précise des segments dans le support.

2. Procédé selon la revendication 1,
**caractérisé en ce**
**que** le modèle de travail dentaire est fabriqué sous la forme d'au moins deux segments adjacents (14, 16, 18, 20, 22, 24), sachant qu'au moins un segment, de préférence chaque segment comprend au moins deux parties de dent ayant une base commune (30, 32, 68).

3. Procédé selon la revendication 1 ou 2,
**caractérisé en ce**
**que** le support (10, 100, 200, 300) utilisé est un élément en forme de plaque avec un côté supérieur et un côté inférieur (36, 38) ainsi que la cavité ou l'évidement (34) ayant un tracé en forme d'arc et étant délimité(e) par les surfaces latérales (52, 54) et qui est équipé du référencement.

4. Procédé selon au moins l'une des revendications précédentes,
**caractérisé en ce**
**que** la cavité (34) est délimitée sur le côté inférieur par une paroi inférieure dans laquelle se trouve une fente longitudinale (40), qui traverse le cas échéant la paroi inférieure, sachant que la base (30, 32, 68) côté fond est formée avec un premier épaulement (64) pour une insertion dans la fente longitudinale.

5. Procédé selon au moins l'une des revendications précédentes,
**caractérisé en ce**
**que** l'élément en forme de plaque comprend sur la face supérieure une première section (82) évidée par rapport à la surface, s'étendant le long de l'évidement (34) et étant en continuité avec ce dernier et que la base (64) est fabriquée de sorte à ce qu'un second épaulement (72) en saillie latérale sort de cette dernière et est adapté à la première section évidée ou à une zone de cette dernière.

6. Procédé selon au moins l'une des revendications précédentes,
**caractérisé en ce**
**qu'**une prothèse dentaire est fabriquée manuellement et/ou à l'aide de procédés CAO/FAO sur la base du modèle de travail dentaire.

7. Plaque de base (10, 100) destinée à recevoir un modèle de dentition (12) constitué de plusieurs segments, sachant que chaque segment comprend une base (68) avec une partie de dent (66) ou plusieurs parties de dent et sachant que la plaque de base comprend un côté supérieur et un côté inférieur (36, 38) ainsi qu'un évidement ou une cavité (34) suivant un arc, tel un arc de dentition, avec des surfaces latérales (52, 54) lesquelles, ou au moins une de leurs surfaces latérales, présentent ou présente un référencement mécanique formant un premier guide pour la base, sachant que la base et la partie de dent sont fabriquées sous forme d'une unité à partir du même matériau,
**caractérisé en ce**
**que** le côté supérieur (36) de la plaque de base (10, 100) présente une zone évidée (82) s'étendant le long de la cavité (34) en tant que deuxième guide pour la base (68) au moyen d'un second épaulement (72) en saillie latérale de la base et adapté à la zone évidée et que, sur la face inférieure, la cavité présente une fente (40) s'étendant le long de la cavité en tant que troisième guide pour la base par engagement d'un premier épaulement (64) en saillie de la base côté fond dans la fente.

8. Plaque de base selon la revendication 7,
**caractérisé en ce**
**que**, vue en coupe, la fente (40) est conçue de préférence en forme de cavité en V ou en U dans la paroi inférieure (35) ou traverse la paroi inférieure.

9. Plaque de base selon la revendication 7 ou 8,
**caractérisé en ce**
**que**, sur la face inférieure, la fente (40) est pontée par au moins une nervure (42,44, 46,48) s'étendant transversalement à la direction longitudinale de la fente.

10. Plaque de base selon au moins l'une des revendications 7 à 9,
**caractérisé en ce**
**que** le premier guide est formé par les surfaces latérales (52, 54) présentant chacune une géométrie ondulée avec des reliefs (56, 58, 60, 62) et des creux s'étendant verticalement par rapport à la face supérieure (36).

11. Plaque de base selon au moins l'une des revendications 7 à 10,
**caractérisé en ce**
**que** l'écart entre les reliefs (56, 58) d'une surface latérale (52) diffère de l'écart entre les reliefs (60, 62) de l'autre surface latérale (54), en particulier que l'écart entre deux reliefs de la surface latérale s'étendant le long de l'arc extérieur est 1,5 à 10 fois, de préférence 2 à 5 fois, plus grande que l'écart entre deux reliefs de la surface latérale opposée s'étendant le long de l'arc intérieur.

12. Plaque de base selon au moins l'une des revendications 7 à 11,
**caractérisé en ce**
**qu'**au moins une nervure (42, 44, 46, 48) est un guide et/ou une fixation pour un articulateur.

13. Équipement dentaire comprenant un modèle de travail dentaire (12) constitué d'au moins deux segments (14, 16, 18, 20, 22) ainsi qu'une plaque de base (10, 100) réceptionnant le modèle de travail dentaire de manière amovible dans un évidement ou une cavité (34), sachant que chaque segment comprend au moins une partie de dent (66) sortant d'une base (68), de préférence au moins deux parties de dent, et que la partie de dent et la base sont fabriqués sous forme d'une unité à partir du même matériau,
**caractérisé en ce**
**que** la cavité (34) comprend une fente (40) s'étendant côté fond, le long de la cavité et que la base comprend un premier épaulement (64) adapté géométriquement à la fente et faisant saillie de la base côté fond, que la plaque de base (10, 100) comprend sur le côté supérieur une zone (82) s'étendant le long de la cavité (34) et en retrait par rapport à sa surface et que la base (68) comprend un second épaulement (72) faisant saillie latéralement et adapté géométriquement à la zone.

14. Équipement dentaire selon la revendication 13,
**caractérisé en ce**
**que** les segments (14, 16, 18, 20, 22) sont disposés dans la cavité (34) en étant bout à bout et que de préférence au moins deux parties de dent sortent d'une base commune.

15. Équipement dentaire selon la revendication 13,
**caractérisé en ce**
**que** les segments (14, 16, 18, 20, 22) insérés dans la plaque de base (10, 100), de préférence fabriqués par procédé FAO, présentent un écart A pour A < 0,1 mm, en particulier A << 0,1 mm.
